(19) Europäisches Patentamt
European Patent Office
Office européen des brevets

(11) EP 0 612 260 B1

(12) **EUROPEAN PATENT SPECIFICATION**

(45) Date of publication and mention
of the grant of the patent:
**27.01.1999 Bulletin 1999/04**

(21) Application number: **92905357.7**

(22) Date of filing: **04.11.1991**

(51) Int. Cl.$^6$: **A61N 5/00**, A61N 5/02

(86) International application number:
**PCT/US91/08136**

(87) International publication number:
**WO 93/08875 (13.05.1993 Gazette 1993/12)**

(54) **HYPERTHERMIA APPARATUS HAVING THREE-DIMENSIONAL FOCUSING**

HYPERTHERMIEGERÄT MIT DREIDIMENSIONALER FOKUSSIERUNG

APPAREIL D'HYPERTHERMIE A REGLAGE TRI-DIMENSIONNEL

(84) Designated Contracting States:
**AT BE CH DE DK ES FR GB GR IT LI LU NL SE**

(43) Date of publication of application:
**31.08.1994 Bulletin 1994/35**

(73) Proprietor:
**BSD MEDICAL CORPORATION**
**Salt Lake City, Utah 84119 (US)**

(72) Inventor: **TURNER, Paul, F.**
**North Salt Lake, UT 84054 (US)**

(74) Representative:
**Grünecker, Kinkeldey,**
**Stockmair & Schwanhäusser**
**Anwaltssozietät**
**Maximilianstrasse 58**
**80538 München (DE)**

(56) References cited:
**EP-A- 0 207 729         EP-A- 0 256 524**
**EP-A- 0 386 246         WO-A-81/02841**
**WO-A-88/03823          DE-A- 2 420 883**
**US-A- 4 572 190         US-A- 4 589 424**
**US-A- 4 638 813         US-A- 4 702 262**
**US-A- 4 815 479         US-A- 4 860 752**
**US-A- 4 934 365**

## Description

Background of the Invention

This invention relates to a hyperthermia apparatus and more particularly to an apparatus having three-dimensional (3-D) focusing capability with combined hyperthermia treatment and noninvasive thermometry capabilities.

Known hyperthermia treatment systems include multiple applicators and multiple temperature sensors for controlling the operation of the hyperthermia system. The multiple applicators utilizing ultrasound or electromagnetic energy in the direct contact operating mode are placed directly upon an elastic cooling belt containing a circulating cooling liquid to carry the heat of hyperthermia treatment away from the surface of the healthy tissue. The temperature sensors are implanted in the normal tissue, in the vicinity of the tumor, as well as within the tumor, Systems involving the placement of temperature sensors within the body are referred to as invasive thermometry systems. Those persons skilled in the art desiring additional information for this system are referred to US-A-4,397,314.

A known instrument for detecting microwave energy and for giving an accurate measurement of the power density thereof is disclosed in US-A-3,919,638. This instrument is substantially unaffected by polarization or modulation of the electromagnetic waves and includes a planar array of parallel connected diode detectors each having a pair of antenna leads forming a dipole antenna. The diode array may include groups of diodes having different antenna lead lengths to detect different frequencies of microwave energy for display by a meter. The meter may be selectively switched between the outputs of the different groups.

Further, the potential use of multiple frequency band radiometry as a means of noninvasive sensing of one-dimensional temperature profiles is presented in an article entitled "Noninvasive Thermometry Using Multiple-Frequency-Band Radiometry: A Feasibility Study:, Stavros D. Prionas and G. M. Hahn, Bioelectromagnetic 6:391-404, 1985 Alan R. Liss, Inc. The article discloses that microwave thermography has been extensively used for the detection of cancerous nodules. Operating frequencies in the range of 1.3 to 6.0 GHz (free space wavelengths in the range of 5 to 23 cm.s) have been employed. At these long wavelengths subcutaneous temperature measurement is possible and detection of superficial tumors in the brain and thyroid is, in principle, feasible.

The article further discloses that a computer tomographic approach using 10 GHz microwaves has been proposed as an alternative to mammographic examination. A self-balancing microwave radiometer for measuring the energy emitted from a heated volume within a single frequency band has been developed at the RCA laboratories. The power spectrum of thermal noise generated by a given temperature depth distribution is governed by Planck's law of blackbody radiation. The frequency spectrum of energy received it the surface of the human body is affected by the frequency dependent attenuation properties of the intervening tissues. Microwave radiometry (the technique of measuring noncoherent electromagnetic energy in the microwave part of the spectrum that is emitted or scattered by the medium under observation) can be used to measure the thermal noise emitted from a heated volume of biological tissue.

This article reports the analysis of the spectral content of this thermal noise and the comparison of the magnitude of the signal to the inherent threshold of noise detectability associated with an ideal microwave radiometer. In the analysis, a one-dimensional temperature distribution model was assumed. In real situations, three-dimensional temperature distributions will be encountered. It is clear that to resolve such a three-dimensional temperature field with a reasonable amount of spatial resolution additional information will be needed. This additional information might be in the form of data acquired using different orientations of a signal receiving aperture or a properly phased array of receiving apertures. An intriguing alternative is to employ a phased array of receiving apertures that coherently detect the signal emanating from a point in space. In either case, well established signal processing algorithms could be used to convert the measured data to reconstruct the temperature distribution.

The use of "Radiometer Receivers for Microwave Thermography" was disclosed in an article of the same title by D. V. Land, University of Glasgow, Glasgow, Great Britain, published in the Microwave Journal, May 1983. A comparison or Dicke radiometer configuration is used. The receiver produces on output from the input switching that is proportional to the difference between the source temperature detected by an antenna and the temperature of an internal reference load or noise generator.

Finally, the application of broad-band correlation techniques to medical microwave thermography was studied and reported in on article entitled "The Thermal and Spatial Resolution of a Broad-Band Correlation Radiometer with Application to Medical Microwave Thermography", Joseph C. Hill et al., IEEE Transactions on Microwave Theory and Techniques, Vol. MTT-33, No. 8, August, 1985.

A prior art (US-A-4,638,813) apparatus as indicated in the precharacterizing part of claim 1 comprises a plurality of antenna groups including two antennae stacked in the direction of the long axis of the body portion surrounded by the plurality of antenna groups. However, these antenna groups are positioned for focusing a received electromagnetic energy to form a target heating spot selectively located within a body portion surrounded by the antenna groups only in a cross section plane of the body portion, meaning the location of the target heating spot can be selected or displaced only within two dimensions.

Another prior art (US 4,798,215) apparatus uses cross-sectional heating energy steering and radiometric EM energy measurement in two dimensions, meaning the cross-section of the body portion.

Another prior art (US-A-4,860,752) apparatus uses an interstitially inserted diode phased array which is inserted directly within the tissues of the body for heating of a tissues within the array. This array configuration also provides only two dimensional steering of the energy with the cross-sectional plane.

Still another prior art (US-A-4,934,365) apparatus uses a pair of concentric ring EM electrodes similar to the apparatus as disclosed in US-A-4,638,813. These two spaced electrodes act as antenna by radiating EM energy in the central region of the body portion. There are also additional fringe electrodes added to provide a radial tapering of the energy at the outer regions of the apparatus with the intent to radius localized hot spots in that region.

The previous annular phased arrays were capable of steering the heating pattern within the patient cross-section (target) with either power or phase steering. This feature was a primary function which resulted from the unique focusing provided by the coherent or at least synchronous EM fields which provided common polarization alignment of the electric field (E-field). The E-field in the target is the mechanism which creates the heating of the tissues.

It is the object of the invention to improve an apparatus as indicated in the precharacterizing part of a claim 1 such that a three-dimensional focusing in a target of a body tissue is achieved without moving the antennas with respect to the long axis position of the body portion.

This object is solved by the features as claimed in claim 1.

Briefly stated the hyperthermia apparatus having 3-D focusing constituting the subject matter of the invention consists of one or more groups of antennae, each group consisting of at least three antennae stacked along the body axis. The power directed to each element of the group or groups of antennae is adjusted in phase and amplitude to focus electromagnetic energy to form a desired heating pattern in a target location for heating a preselected target in a body anywhere within the boundaries of the apparatus.

The present invention uses it least an antenna group of at least three antennae stacked along the E-field direction and aligned with the long axis of the target. Thus, in a dipole configuration, the dipole lengths are aligned with the long axis of the target around which it is placed. This arrangement permits the heating fields to be not only focused and steered along the patient's cross-section but also along the long axis of the target.

An advantage of the 3-D hyperthermia apparatus is that in its simplest form (a single group), it allows the phase of the antennae to be set to provide a desired heating length. Another advantage is that a plurality of groups can be utilized to provide deep target heating capability. Yet another advantage is the adjustment of the phase and power of each group's elements to regulate the position of the heating region in the target.

Brief Description of the Drawings

Other objects and features of the invention will become more readily apparent from the following detailed description when read in conjunction with the accompanying drawings, in which:

FIGURE 1 is a schematic view in block form of the three-dimensional (3-D) hyperthermia system constituting the subject matter of the invention;

FIGURE 2 is a view showing a cylindrical arrangement of groups of dipole antennae forming an electromagnetic applicator of the 3-D hyperthermia system;

FIGURE 3 is a partial schematic view showing in block form a first power connector arrangement for the dipoles of at least two dipole antenna groups;

FIGURE 4 is a partial schematic view showing in block form a second power connector arrangement for the dipoles of at least two dipole antenna groups;

FIGURE 5 is a view including a partial schematic in block diagram showing a first power connector arrangement for the groups of dipole antennae of FIGURE 2 and a plan view of the applicator;

FIGURE 6 is a partial schematic view showing in block form a second power connector arrangement for the eight groups of dipole antennae of FIGURE 2;

FIGURE 7 is a partial schematic view showing in block form a third power connector arrangement for the eight groups of dipole antennae of FIGURE 2;

FIGURE 8 is a schematic view showing in block form a fourth power connector arrangement for the eight groups of dipole antennae of FIGURE 2.

FIGURES 9-11 are views showing the paths of travel for various phase settings for the dipoles of the groups of antennae of FIGURE 2 of which only two opposing groups are shown as being representative of the groups.

FIGURE 12 is a view showing the addition of the radiometric receiver to the first power connector arrangement of FIGURE 5.

Description of the Preferred Embodiments

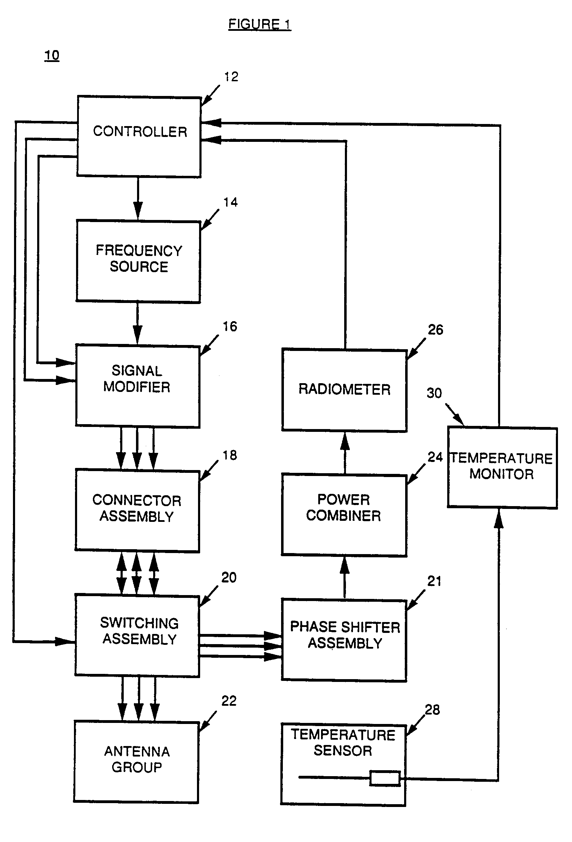

The three-dimensional (3-D) focusing hyperthermia system 10 (FIG. 1) includes a controller 12 connected to a frequency source of electromagnetic energy 14. The controller 12 is connected to a signal modifier 16 which includes as more particularly described hereinafter a power splitter and/or a plurality of amplifiers and phase shifters. The signal modifier 16 enables the modification of the phase and power amplitude of a multiple channel RF power system. A connector assembly 18 connects a switching assembly 20 to the signal modifier 16. The switching assembly 20 connects the connector assembly 18 to the antenna group or antenna groups 22 forming the applicator for the apparatus.

The radiometric circuit includes a phase shifting assembly 21 connected to the switching assembly 20 for directing the received signals generated from the body (blackbody radiation) to a power combiner 24 with equal or non-equal relative phase shift. A radiometer 26 is connected to the power combiner 24. The phase shift of the received signal enables the point of primary detection to be shifted to different regions within the body. This phase shifter may also include switches to eliminate or reduce the transfer of energy from certain ones of the antenna (dipole) elements. In this way it would enable the receipt of energy to favor certain dipole paths to a measure of more superficial tissue temperatures. Further, by activating at times only portions of these phase shifter channels, the tissue temperature around the surface region of the target tissue is measured noninvasively. The temperature measurements are made using the power detection capability of the radiometer. The radiometer 26 has its output connected to the controller 12 for controlling system operation. Power levels and phase of the signal modifier may be adjusted either by an operator or by a control loop to assure that the target tissue is maintained at the selected temperature and the non-target tissue is not overheated.

A temperature sensor assembly 28 of one or more temperature seniors are placed invasively into the tissue for measuring the temperature of the tissues being heated. Finally, a temperature monitor 30 is connected between the temperature sensor 28 and controller 12 for monitoring the temperature output signals of the temperature sensor assembly for the controller 12 to complete the system. Those persons skilled in the art are referred to US-A-4,798,215 for additional information concerning the controller, radiometric circuit and temperature sensing circuit.

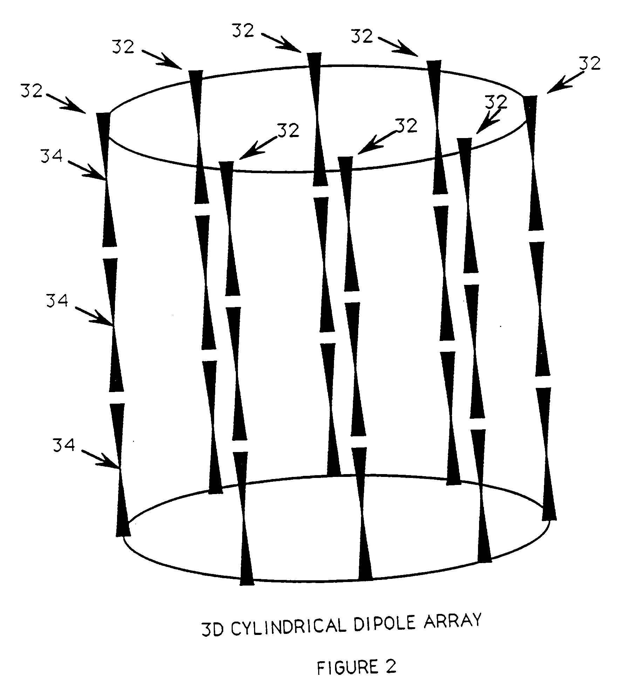

Referring now to FIGURE 2, the 3-D cylindrical dipole array consists of a plurality of groups 32 of antennae 34. Each group of antennae 32 includes at least three antennae 34 stacked end to end along the direction of the E-field polarization axis.

Numerical modeling studies show that little improvement is obtained when the number of antenna groups exceed eight. As the number of groups in the cylindrical shape decreases to fewer than eight, the depth of penetration begins to decrease. Nevertheless, the invention is not limited to eight antenna groups and any number of groups above or below eight are to be considered to be included in the invention.

It will be appreciated that the three stacked dipoles could be extended to include more dipoles and the phase of each could be controlled to provide proper phase alignment for target selection. If desired in connection with very complex arrays, a computer can be programmed in the controller to select the proper phase and power for each antenna. Although, for purposes of description and not by way of limitation, dipole type antennae are referred to throughout the description, the types of antennae may include patch, metal strips, metallic waveguide, dielectric waveguides, resonant cavities, coaxial antennae TEM mode horn types to name but a few types which can be used in practicing the invention.

The dipole antennae are preferably made of tapered metallic conductive strips with the feed located midway between the two strips forming the dipole. The taper is increased outwardly from the central feed point to increase the frequency bandwidth and the near field energy along the region of the tips of the dipoles. The length of the dipoles should be determined in light of the operating frequency to prevent loss of energy.

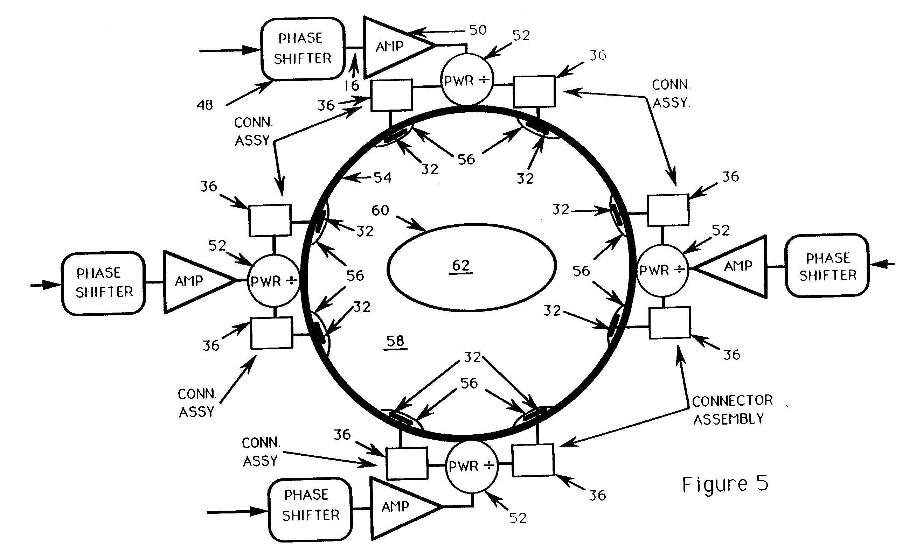

As shown in FIGURE 5, these dipole groups 32 are formed normally along the inside wall of a clear plastic or dielectric cylinder 54 using well-known adhesives or metal deposition processes. A thin patch of dielectric coating material 56 is used to cover the antennae groups 32. A bolus 58, can be formed with the cylinder by attaching a membrane 60 having sealed ends to the cylinder 54. A fluid input/output valve can be mounted in the cylinder 54 for inflating the bolus with the fluid. The inflated bolus defines the body area 62, provides tissue cooling, energy confinement, and improves antenna group coupling to the tissue. The fluid is a high dielectric low loss media such as deionized water.

The bolus has been included in other applicators. Nevertheless, in practicing this invention it is important to take into account the dielectric characteristics of the bolus region and the body as well when planning the activation phase of the individual antenna elements 34, FIG. 2, of antenna groups 32.

The normal operating frequency range for this system for the torso portion of an adult is between about 50 to 1000 MHz. It is most useful between 60 to 220 MHz where the penetration characteristics of the body are deeper. The lower frequencies however have larger wavelengths and limit the precision to which the tissue can be selectively heated. The phase of the dipoles determines the location of a focal point within the body. As dipoles have a wide pattern, the focal point is where the same phases exist in the overlapping beams. At the other points in the overlapping beams the phase is different and the energy partially cancels. The typical size of the focal point defining the target in the body is about 30 to 50% of the wavelength within the tissue into which the electromagnetic energy is directed. The wavelength is inversely proportional to frequency. For example, at 100 MHz the wavelength in the high water tissues of the body is 27 cm.s and in deionized water is 30 cm.s. This similarity simplifies a rough estimate for planning the required phase activation for a certain focal zone because of the similar size. For example, the focal size of the 100 MHz EM energy within the high water content tissue is between 9 to 14 cm.s in diameter.

From the above, it is readily apparent that if the operation frequency is changed to 200 MHz the focal diameter would change to about 4.5 to 7 cm.s for a wavelength of 14 cm.s in the high water tissues. While changing the frequency provides higher precision for the focusing, the penetration depth is less than that for 100 MHz. For 100 MHz operation the dipole, if the dipole length is one-half of a wavelength within the water media, should be an efficient radiator having a length of about 15 cm.s; if a dielectric coating is placed over the dipoles the operating frequency may need to be increased.

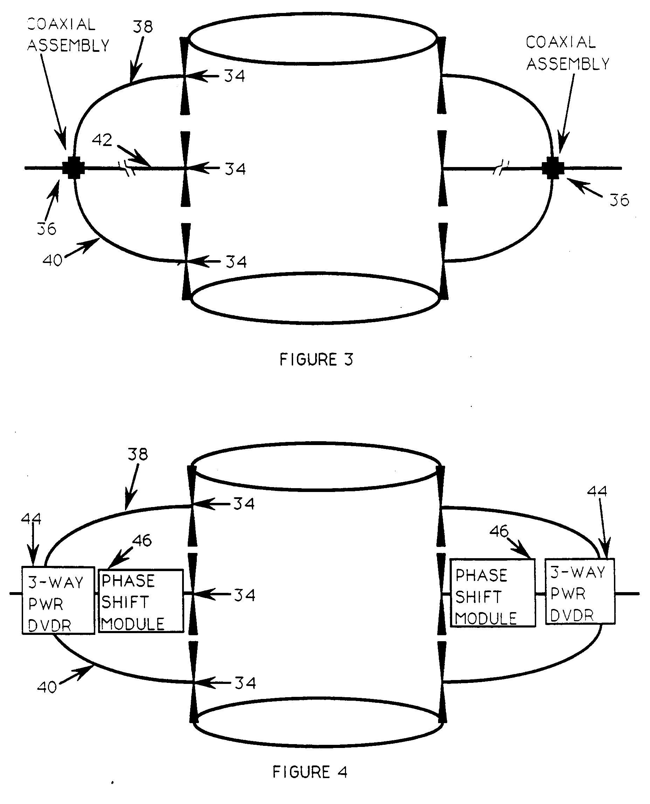

A first embodiment of the connector assembly 18 for each antenna group 32 is shown in FIGURE 3. As the connectors of the connector assembly are identical only two of the antenna groups 32 are shown and one described for clarity and brevity of the description. Thus, each connector 36 includes a coaxial cable connected between the signal modifier 16 (FIG. 1) and a tee. The tee 36 is connected to coaxial cables 38, 40 and 42. The coaxial cables 38 and 40 have substantially identical lengths for providing power at the same phases to the end antennae 34. While coaxial cable 42 has a longer length to compensate for the shorter distance from the central dipole and the central focus point.

In a second embodiment of the connector assembly 18 (FIG. 4), a power divider 44 is connected to the signal modifier 16 (FIG. 1). Coaxial cables 38 and 40 interconnect the outer power divider outputs to the outer antenna elements or dipoles of the antenna groups 32. A phase shift module 46 interconnects the center output of the power divider to the center antenna elements or dipoles of antenna groups 32. Again, if the cables 38 and 40 have the same length, the phase shift module can be set to provide an operation equivalent to the first embodiment; however, the phase of the phase shift module is variable for adjusting the size of the heating field along the long body axis. This is the result of the phase focusing within the primary target zone and the destructive phase cancelling which occurs from the different phase in the regions displaced from the phase focus. This destructive or cancelling phase is the result of the different path lengths to the non-target point resulting in the partial cancelling of the energy within these off target areas. When the control antennae radiated phase to the center is set to be different from the outer antennae, the heating length is increased. This also causes a reduced intensity of the power to the center tissue. This can cause higher reductions in the surface heating levels as compared to that within the central region, and is most effective when the phase shifts of the central antennae are caused to lead the maximum focus values of phase for the other antennae. Thus, this embodiment represents the full steering capability of the system, and the variable used to optimize a treatment utilizes proper selection of the phase and power to each dipole at a selected frequency. When eight groups of three dipoles are used it is preferred that they be activated by a signal modifier which includes power splitters, phase shifters and a four, eight, sixteen, or twenty-four channel amplifier.

Referring to FIGURE 5, when the signal modifier 16 includes a four channel amplifier, a four-way power divider is generally used to divide the power from the controller 12. The signal modifier 16 includes four circuits each including a phase shifter 48, amplifier 50 and a two-way power divider 52. The phase shifter 48 is connected to the power divider or power source for controlling the phase of the power applied to an amplifier 50. Amplifier 50 provides the increase in signal power (gain) necessary for the pair of adjacent antenna or dipole groups 32. While the power divider 52 divides the amplified power for input to the connector assemblies 36 connected to each antenna or dipole group 32. The connector assemblies are those described in connection with FIGURES 3 and 4.

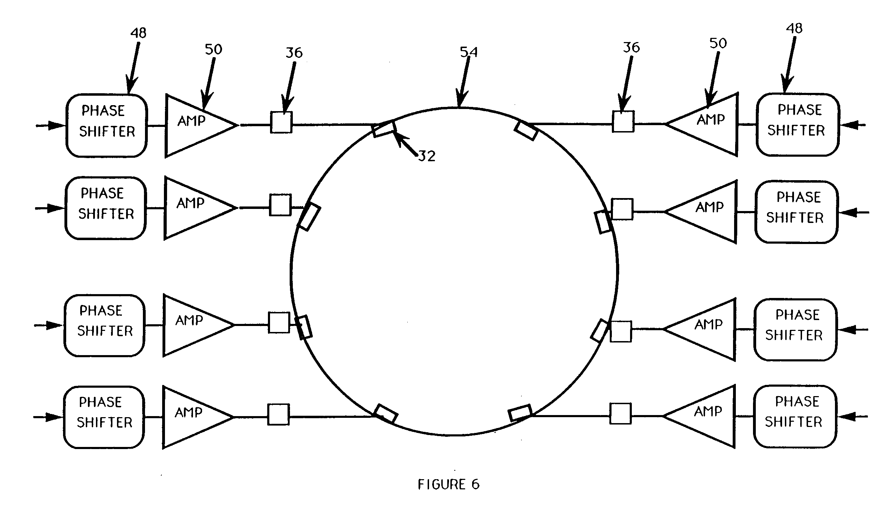

If an eight channel amplifier is used (FIG. 6), then the 4 two-way power dividers 52 of FIGURE 5 are removed, and each antenna group 32 is connected directly to the connector assembly 36. Thus, the central dipoles phase would still be settable by either cable length changes (FIG. 3) or by adjustable phase shifters (FIG. 4).

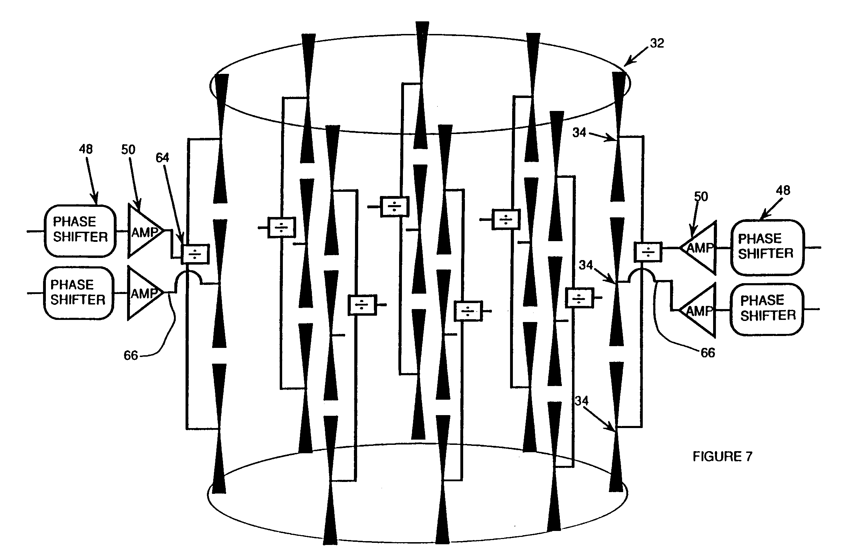

When a sixteen channel amplifier is used (FIG. 7), the connector assembly 36 used to connect the three dipoles of each group would not be used. In lieu thereof each of the center dipoles of the eight groups 32 are connected directly to an amplifier of the sixteen channel amplifier of the signal modifier 16. The outer dipoles are connected in groups of two through two-way dividers 64 to the remaining eight amplifiers 50 of the sixteen channel amplifier. The phase of the respective dipoles is set by the phase shifters 48 connected to the sixteen channel amplifiers. It will be appreciated by those skilled in the art that this arrangement will not provide long axis steering unless the phase to the end antennae are made to be different for grouped pairs.

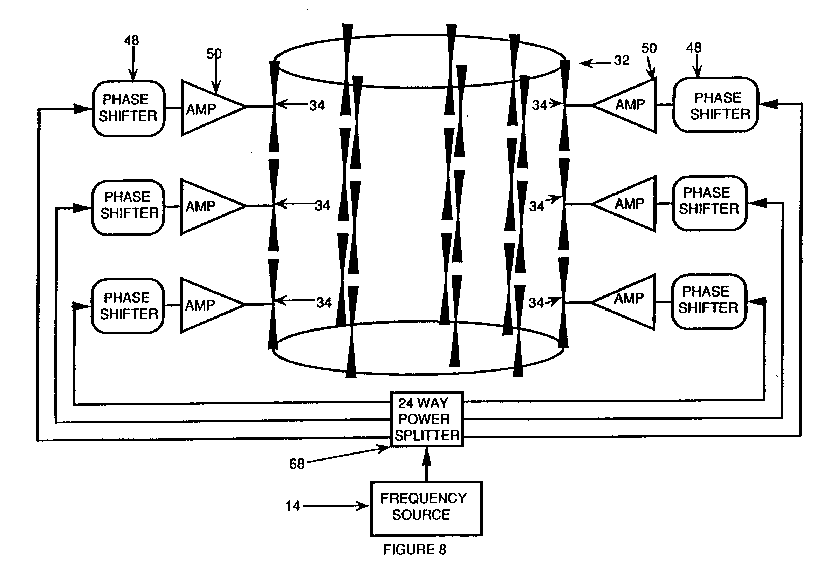

For the 24 amplifier system (FIG. 8), the connector assembly 18 is omitted. A 24-way power splitter 68 is connected to the controller 14 for dividing the frequency source, and each signal modifier circuit 16 has a phase shifter 48 and an amplifier 50 connected to one of the 24 dipoles 34 of the eight groups 32. For deepest target heating the frequency of all antennae should be the same. However, different or changing frequencies, powers, or phases can be varied to obtain desired heating patterns. The change in frequencies will change surface heating. This is desirable when the target is located along the surface region. The control of these parameters would enable optimal treatments with these EM heating phased arrays.

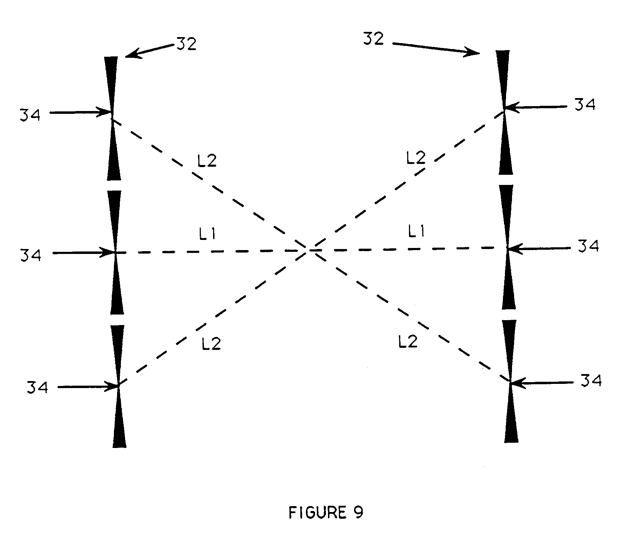

Referring now to FIGURES 9-11, FIGURE 9 shows the path of travel for the EM radiation traveling from the dipoles toward the central heating zone. For clarity only two opposing groups 32 of antennas or dipoles 34 are shown as typical of the preferred eight groups. The lengths of the paths are designated by the distances $L_1$ and $L_2$. Path $L_1$ represents the path traveled by the energy leaving the central dipole for the central focus. $L_2$ represents the corresponding path lengths for the energy leaving the outer dipoles for the central focus. To accomplish maximal central energy focus and minimal long axis heating the activation phase of the central antennae should lag that of the end antennae by the difference of the equivalent phase delays of the paths $L_1$ and $L_2$. If $L_1$ and $L_2$ were assumed to be totally within a water bolus, at 100 MHz the water wavelength is 30 cm.s. Thus, the difference of $L_2$-$L_1$ could be divided by the wavelength and multiplied by 360 degrees to find the phase lag setting for the central antennae.

$$\text{Central Phase Setting} = (L_2 - L_1)^* (360)/\text{wavelength, degrees phase lag.}$$

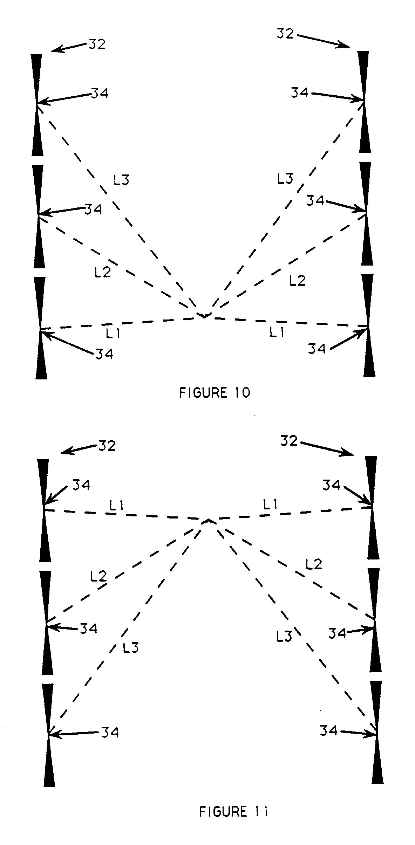

FIGURE 10 shows that if the phase of each antenna 34 is adjusted to obtain the same radiated phase at an offset position along the central axis that the radiated phase of paths $L_1$, $L_2$, and $L_3$ must be adjusted to arrive in phase at the off-set target. The result is that if the phase has been optimized for the off-set central focal zone that the heating will become more focused in the offset zone represented by FIGURE 10. Thus, to steer the heating field focal zone along the long axis as shown in FIGURE 11, it is necessary to adjust the phase of the power of each antenna to obtain the same radiated phase at an off-set position to the central zone along the long axis. This is in addition to steering the focal point along the cross-sectional axis as previously described in the parent application.

Each of the above described heating configurations, can be operated with a radiometric non-invasive temperature sensor by the addition of coaxial switches at the common coaxial tee locations. This would be at the tee of FIGURE 3 and the power divider 44 of FIGURE 4. The transmit system of FIGURE 8 has no power splitting devices following the amplifiers, so there would be included 24 radiometric switches and phase shifters in the phase shifters assembly 21 to incorporate the radiometric non-invasive thermometry. Thus, for this system, a 24 port summing power combiner 24 is required. The transmit systems of FIGURES 5, 6, and 7 have 4, 8, and 16 splitter or tee junctions to the amplifiers so a corresponding number of switches are required for each to connect to the combiner. The phase shifter assembly 21 operates with the phase fixed, controlled, or adjustable to enable the phase focus of radiometric reception to be set at common locations within the body being heated. The radiometric mode is enabled by the closure of at least some of the switches within the phase shifter assembly 21 to the radiometer. The transmitted power is interrupted by the switches to avoid interference to the sensitive radiometric receivers.

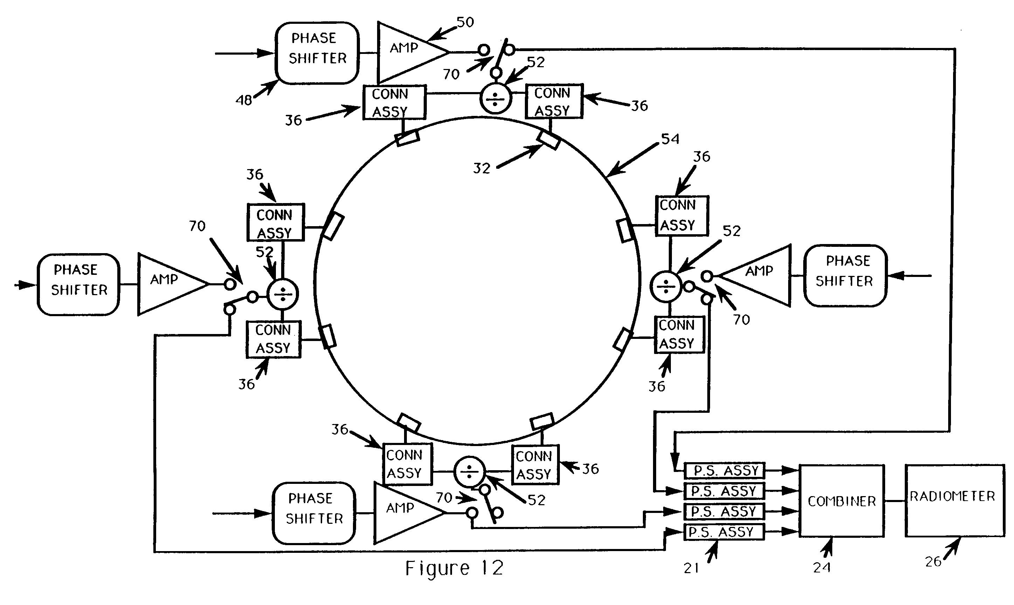

FIGURE 12 shows the addition of the radiometric receiver channels to the four channel system configuration of FIGURE 5. As shown in FIGURE 12 the switch 70 is a single pole two contact switch for switching between the heating power circuit and the radiometric circuit. The switches are placed between the amplifiers 50 and the power dividers 52 for the antenna groups 32 to avoid common phase shifting devices for both the transmit and received modes, unless these devices are very low loss such as coaxial cable. Otherwise the added noise of a hot high powers phase shifter may interfere with the accurate noninvasive temperature measurement.

Some of the switching functions of the switching assembly 20 may be incorporated with the phase shifter assembly 21 to deactivate the connection of certain of the antenna group 32 (within the overall antenna group 22) to the power combiner to cause selective non-invasive tissue temperature measurement in particular surface regions underlying the connected antenna groups 32. This flexibility of connection paths could be within the switching assembly 20 or within the phase shifter assembly 21. Such switching is under the control of the controller 12 as indicated by the lead between the controller and the switching assembly of FIGURE 1. Similarly, the controller may operate through the switching assembly 20 to the phase shifter 21 for controlling the phase shifter assembly.

## Claims

1. A hyperthermia apparatus having a focusing capability comprising:

   a first means (14) for generating electromagnetic energy at preselected frequencies, phase and power levels;

   a second means (18) connected to the first means (14) for receiving the electromagnetic energy from the first means and distributing it to a third means (22), said second means (18) including means for adjusting relative phases of electromagnetic energy distributed to portions of the third means (22); and

   the third means (22) including a plurality of antenna groups (32) is connected to the second means (18,20) and positioned for focusing the received electromagnetic energy to form a target heating spot selectively located within a body portion (62) surrounded by the third means
   **characterized in that**

   each antenna group (32) includes at least three antennae (34) stacked in the direction of the long axis of the

EP 0 612 260 B1

body portion (62) surrounded by the plurality of antenna groups (32), each of the at least three antennae (34) being connected to the second means (18,20) to receive electromagnetic energy of selected phase therefrom for focusing spot size or location in the direction of the long axis of the body portion (62) whereby three-dimensional location of the target heating spot is provided.

2. Apparatus according to claim 1 wherein the antennae (34) of the plurality of antenna groups (32) are selected from the group consisting of dipole, patch, metal strip, metallic waveguide, dielectric wave guides, resonant cavities, coaxial antenna, and TEM mode horns.

3. Apparatus according to claim 2 wherein the plurality of antenna groups includes eight antenna groups (32), each group including three dipole antennae (34) stacked end to end.

4. Apparatus according to any of clams 1 to 3 wherein the first means (14) for generating electromagnetic energy at preselected frequencies, phase and power levels includes a power splitting means (68) for dividing electromagnetic energy for a plurality of channels, a plurality of power amplifying means (50) connected to the power splitting means (68) for setting the power gain for each channel, and a corresponding plurality of phase shifting means (48) connected to the power amplifying means (68) for setting the power and phase for each channel.

5. Apparatus according to claim 4 wherein the plurality of power amplifying means (58) of the first means (14) is a four channel amplifier, the power splitting means (68) is a four-way power splitter, and further including a power divider (52) connected to each amplifier (50) for dividing the power between the antennae groups (32) or the third means (22).

6. Apparatus according to claim 4 wherein the plurality of power amplifying means (50) of the first means (14) is an eight channel amplifier and the power splitting means (68) is an eight-way power splitting means connected to the eight channel amplifier.

7. Apparatus according to claim 4 wherein the plurality of power amplifying means (50) of the first means (14) is a twenty-four channel amplifier, and the power splitting means is a 24-way power splitter (68).

8. Apparatus according to claim 4 wherein the plurality of power amplifying means (50) is a sixteen channel amplifier, the power splitting means (68) is a sixteen-way power splitter, and wherein eight antenna groups (32) for the third means (22) and each include three dipole antennae (34) stacked end to end, and wherein eight amplifiers (50) of the sixteen channel amplifier are connected directly to the centrally located antenna of the eight antenna group (32), and the remaining eight amplifiers are connected to eight two-way power dividing means (64), each of the two-way power dividing means being connected to the opposing end dipoles of an antenna group (32), and the phase of the opposing end antennae for each of the antenna groups being made different that the phase of the centrally located antennae of that group for the adjustment of the heating spot size in the direction of the long axis.

9. Apparatus according to any one of claims 1 to 8 further including a radiometric means (26) connected to the third means (22) for measuring energy radiating from the body portion (62) surrounded by the third means for determining noninvasively the temperature of the target heating spot and its surrounding media.

10. A hyperthermia apparatus according to claim 9 wherein the radiometric means includes a switching means (20) connected between the second (18) and third means (22) for connecting the third means (22) selectively to the second means (18) for receiving electromagnetic energy and to the radiometric means (26) for receiving the energy radiating from the body portion (62) through the third means (22), a radiant energy phase assembly (21) connected to the switching means (20) for adjusting the phase of the radiant energy received, a combiner (24) connected to the radiant energy phase assembly (21) for combining the radiant energy signals received, and a radiometer (26) for measuring the radiant energy signals from the combiner (24) for determining the temperature of the target heating spot.

11. Apparatus according to any one of claims 1 to 10 wherein the third means (22) includes a plurality of antenna groups (32) mounted upon a tube (54) of dielectric material, and further including a temperature sensor (28) adapted to be inserted into the heated area for sensing the temperature of the heated target, and temperature monitor means (30) coupled to the temperature sensor (28) for monitoring the sensed temperature of the temperature sensor and supplying signals to the first means (14) to control operation of the first means.

7

**12.** Apparatus according to any one of claims 1 to 11 including a control means (12) connected to the first means (14) for controlling the frequency phase, and power levels of the output of the first means (14).

**13.** Apparatus according to any one of claims 9 to 12 further including a temperature monitoring means connected to the third means (22) for monitoring the temperature of the target heating spot and a control means (12) connected to the radiometer means (26) and temperature monitoring means and to the first means (14) for controlling phase and power level output of the first means (14) responsive to the outputs of the radiometer means (26) and temperature monitoring means.

**14.** Apparatus according to any one of claims 1 to 13 wherein the second means (18) includes an electromagnetic energy connector means (36,38,40,42) connecting the electromagnetic energy received from the first means (14) to the third mans (22), the means in said electromagnetic energy conductor means (36,.. .42) for adjusting the phases of electromagnetic energy distributed to portions of the third means (22) including an energy splitting means (36) for splitting the energy received from the first means (14), and a plurality of electromagnetic energy conductors (38,40,42) connected between the energy splitting means (36) and the third means (22) and having preselected lengths for producing energy outputs having phases corresponding to the lengths of the plurality of electromagnetic energy conductors (38,40,42) for use by the third means (22) for focusing the electromagnetic energy to form a target heating spot.

**15.** Apparatus according to claim 14 wherein a phase shifting module (46) is connected to the energy splitting means (36,44) intermediate at least one of the plurality of electromagnetic energy conductors (38,40,42) for setting the phase of the energy output of said at least one of the plurality of electromagnetic conductors.

**Patentansprüche**

**1.** Hyperthermiegerät mit Fokussierung mit:

einer ersten Einrichtung (14) zum Erzeugen elektromagnetischer Energie bei ausgewählten Frequenzen, Phasen und Leistungspegeln;

einer mit der ersten Einrichtung (14) verbundenen zweiten Einrichtung (18) zum Empfangen der elektromagnetischen Energie von der ersten Einrichtung und zum Verteilen dieser an eine dritte Einrichtung (22), wobei die zweite Einrichtung (18) eine Einrichtung zum Einstellen relativer Phasen der elektromagnetischen Energie umfaßt, die an Teile der dritten Einrichtung (22) verteilt wird, und wobei

die dritte Einrichtung (22), die eine Vielzahl von Antennengruppen (32) umfaßt, mit der zweiten Einrichtung (18,20) verbunden und zum Fokussieren der empfangenen elektromagnetischen Energie angeordnet ist, um einen Zielwärmefleck zu bilden, der selektiv innerhalb eines Körperteils (62) lokalisiert wird, der von der dritten Einrichtung umgeben ist;
**dadurch gekennzeichnet,** daß

jede Antennengruppe (32) mindestens drei Antennen (34) umfaßt, die in Richtung der Längsachse des Körperteils (62) übereinander angeordnet sind, der von der Vielzahl der Antennengruppen (32) umgeben ist, wobei jede der mindestens drei Antennen (34) mit der zweiten Einrichtung (18,20) verbunden ist, um elektromagnetische Energie ausgewählter Phase von dieser zum Fokussieren der Größe oder des Ortes des Flecks in Richtung der Längsachse des Körperteils (62) zu empfangen, wodurch die dreidimensionale Lokalisierung des Zielwärmeflecks bewirkt wird.

**2.** Hyperthermiegerät nach Anspruch 1, wobei die Antennen (34) der Vielzahl von Antennengruppen (32) aus der Gruppe ausgewählt sind, die aus Dipolen, Flächen, Metallstreifen, metallischen Wellenleitern, dielektrischen Wellenleitern, Resonanzhohlräumen, Koaxialantennen und TEM-Mode-Hörnern besteht.

**3.** Hyperthermiegerät nach Anspruch 2, wobei die Vielzahl von Antennengruppen acht Antennengruppen (32) umfaßt, wobei jede Gruppe drei Dipolantennen (34) umfaßt, die von Ende zu Ende übereinander angeordnet sind.

**4.** Hyperthermiegerät nach einem der Ansprüche 1 bis 3, wobei die erste Einrichtung (14) zum Erzeugen von elektromagnetischer Energie bei ausgewählten Frequenzen, Phasen und Leistungspegeln eine Leistungsteilereinrichtung (68) zum Teilen der elektromagnetischen Energie auf eine Vielzahl von Kanälen, eine Vielzahl von Leistungsverstärkereinrichtungen (50), die mit der Leistungsteilereinrichtung (68) zum Einstellen der Leistungsverstärkung für jeden Kanal verbunden sind, und eine zugehörige Vielzahl von Phasenschiebereinrichtungen (48) umfaßt, die mit den Leistungsverstärkereinrichtungen (68) zum Einstellen der Leistung und Phase für jeden Kanal verbunden sind.

5. Hyperthermiegerät nach Anspruch 4, wobei die Vielzahl von Leistungsverstärkereinrichtungen (58) der ersten Einrichtung (14) ein Vierkanalverstärker ist, die Leistungsteilereinrichtung (68) ein Vier-Wege-Leistungsteiler ist und außerdem einen Leistungsteiler (52) umfaßt, der mit jedem Verstärker (50) zum Teilen der Leistung zwischen den Antennengruppen (32) oder der dritten Einrichtung (22) verbunden ist.

6. Hyperthermiegerät nach Anspruch 4, wobei die Vielzahl von Leistungsverstärkungseinrichtungen (50) der ersten Einrichtung (14) ein Acht-Kanal-Verstärker und die Leistungsteilereinrichtung (68) eine Acht-Weg-Leistungsteilereinrichtung ist, die mit dem Acht-Kanal-Verstärker verbunden ist.

7. Hyperthermiegerät nach Anspruch 4, wobei die Vielzahl von Leistungsverstärkungseinrichtungen (50) der ersten Einrichtung (14) ein 24-Kanal-Verstärker ist und die Leistungsteilereinrichtung ein 24-Weg-Leistungsteiler (68) ist.

8. Hyperthermiegerät nach Anspruch 4, wobei die Vielzahl von Leistungsverstärkungseinrichtungen (50) ein Sechzehn-Kanal-Verstärker ist, die Leistungsteilereinrichtung (68) ein Sechzehn-Weg-Leistungsteiler ist, und wobei acht Antennengruppen (32) für die dritte Einrichtung (22) und jede drei Dipolantennen (34) umfaßt, die jeweils von Ende zu Ende übereinander angeordnet sind, und wobei acht Verstärker (50) des Sechzehn-Kanal-Verstärkers unmittelbar mit der zentral angeordneten Antenne der acht Antennengruppen (32) und die übrigen acht Verstärker mit acht Zwei-Weg-Leistungsteilereinrichtungen (64) verbunden sind, wobei jede der Zwei-Weg-Leistungsteilereinrichtungen mit gegenüberliegenden Enddipolen einer Antennengruppe (32) verbunden ist, und die Phase der gegenüberliegenden Endantennen für jede der Antennengruppen derart unterschiedlich gemacht ist, daß die Phase der zentral angeordneten Antennen der Gruppe zur Einstellung der Wärmefleckgröße in Richtung der Längsachse dient.

9. Hyperthermiegerät nach einem der Ansprüche 1 bis 8, das außerdem eine radiometrische Einrichtung (26) umfaßt, die mit der dritten Einrichtung (22) zum Messen der von dem Körperteil (62) abgestrahlten Energie verbunden ist, der von der dritten Einrichtung zum nichtinvasiven Bestimmen der Temperatur des Zielwärmeflecks und seines umgebenden Mediums umgeben ist.

10. Hyperthermiegerät nach Anspruch 9, wobei die radiometrische Einrichtung eine zwischen die zweite (18) und die dritte Einrichtung (22) verbundene Schaltereinrichtung (20) zum Verbinden der dritten Einrichtung (22) wahlweise mit der zweiten Einrichtung (18) zum Empfangen elektromagnetischer Energie und mit der radiometrischen Einrichtung (26) zum Empfangen der von dem Körperteil (62) abgestrahlten Energie über die dritte Einrichtung (22), eine Abstrahlungsenergie-Phasenanordnung (21), die mit der Schaltereinrichtung (20) zum Einstellen der Phase der empfangenen abgestrahlten Energie verbunden ist, einen mit der Abstrahlungsenergie-Phasenanordnung (21) verbundenen Kombinierer (24) zum Kombinieren der empfangenen Abstrahlungsenergiesignale und ein Radiometer (26) zum Messen der Abstrahlungsenergiesignale von dem Kombinierer (24) zum Bestimmen der Temperatur des Zielwärmeflecks umfaßt.

11. Hyperthermiegerät nach einem der Ansprüche 1 bis 10, wobei die dritte Einrichtung (22) eine Vielzahl von Antennengruppen (32) umfaßt, die auf einem Rohr (54) aus dielektrischem Material gehalten sind, sowie außerdem einen Temperaturfühler (28) umfaßt, der in den erwärmten Bereich zum Erfassen der Temperatur des erwärmten Ziels einsetzbar ist, und eine Temperaturüberwachungseinrichtung (30) mit dem Temperaturfühler (28) zum Überwachen der erfaßten Temperatur des Temperatursensors gekoppelt ist und Signale an die erste Einrichtung (14) zum Steuern der Arbeitsweise der ersten Einrichtung zuführt.

12. Hyperthermiegerät nach einem der Ansprüche 1 bis 11, das eine Steuereinrichtung (12) umfaßt, die mit der ersten Einrichtung (14) zum Steuern der Frequenz, Phase und Leistungspegel des Ausgangs der ersten Einrichtung (14) verbunden ist.

13. Hyperthermiegerät nach einem der Ansprüche 9 bis 12, das außerdem eine mit der dritten Einrichtung (22) verbundene Temperaturüberwachungseinrichtung zum Überwachen der Temperatur des Zielwärmeflecks sowie eine mit der Radiometereinrichtung (26) und der Temperaturüberwachungseinrichtung sowie der ersten Einrichtung (14) verbundene Steuereinrichtung (12) zum Steuern von Phase und Leistungspegel der Ausgangsgröße der ersten Einrichtung (14) umfaßt, die auf die Ausgangssignale der Radiometereinrichtung (26) und der Temperaturüberwachungseinrichtung anspricht.

**14.** Hyperthermiegerät nach einem der Ansprüche 1 bis 13, wobei die zweite Einrichtung (18) eine Verbindungseinrichtung (36, 38, 40, 42) für die elektromagnetische Energie umfaßt, die die von der ersten Einrichtung (14) empfangene elektromagnetische Energie an die dritte Einrichtung (22) weitergibt, wobei die Einrichtung in der Verbindungseinrichtung (36, ... 42) für die elektromagnetische Energie zum Einstellen der Phasen der an die Teile der dritten Einrichtung (22) verteilten elektromagnetischen Energie eine Energieteilereinrichtung (36) zum Teilen der von der ersten Einrichtung (14) empfangenen Energie umfaßt, und eine Vielzahl von Leitern (38, 40, 42) für elektromagnetische Energie zwischen der Energieteilereinrichtung (36) und der dritten Einrichtung (22) verbunden sind und vorgewählte Längen zum Erzeugen von Energieausgängen haben, die Phasen entsprechend der Längen der Vielzahl von Leitern (38, 40, 42) für die elektromagnetische Energie haben, die von der dritten Einrichtung (22) zum Fokussieren der elektromagnetischen Energie zum Bilden eines Zielwärmeflecks benutzt werden.

**15.** Hyperthermiegerät nach Anspruch 14, wobei ein Phasenschiebermodul (46) mit der Energieteilereinrichtung (36, 44) zwischen mindestens einen der Vielzahl von Leitern (38, 40, 42) für die elektromagnetische Energie zum Einstellen der Phase des Energieausgangs des mindestens einen der Vielzahl von elektromagnetischen Leitern geschaltet ist.

**Revendications**

**1.** Appareil d'hyperthermie ayant une capacité de focalisation, comprenant :

des premiers moyens (14) pour générer de l'énergie électromagnétique à des fréquences, à une phase et à des niveaux de puissance présélectionnés ;
des seconds moyens (18) connectés aux premiers moyens (14) pour recevoir l'énergie électromagnétique provenant des premiers moyens et la distribuer à des troisièmes moyens (22), lesdits seconds moyens (18) incluant des moyens pour ajuster les phases relatives de l'énergie électromagnétique distribuée à des parties des troisièmes moyens (22) ; et
les troisièmes moyens (22), incluant une pluralité de groupes d'antennes (32), sont connectés aux seconds moyens (18, 20) et sont positionnés pour focaliser l'énergie électromagnétique reçue afin de constituer un point chauffant formant cible sélectivement positionné au sein d'une partie de corps (62) entourée par les troisièmes moyens
caractérisé en ce que
chaque groupe d'antennes (32) inclut au moins trois antennes (34) empilées selon la direction de l'axe long de la partie de corps (62) entourée par la pluralité de groupes d'antennes (32), chacune des au moins trois antennes (34) étant connectée aux seconds moyens (18, 20) pour recevoir l'énergie électromagnétique ayant sa phase sélectionnée par ceux-ci afin de focaliser la taille ou la position du point selon la direction de l'axe long de la partie de corps (62), de sorte que la position tridimensionnelle du point chauffant formant cible est obtenue.

**2.** Appareil selon la revendication 1, dans lequel les antennes (34) de la pluralité de groupes d'antennes (32) sont sélectionnées parmi le groupe constitué d'un dipôle, d'un dicorde, d'un ruban métallique, d'un guide d'ondes métallique, de guides d'ondes diélectriques, de cavités résonnantes, d'une antenne coaxiale et de cornets en mode transversal électromagnétique (TEM).

**3.** Appareil selon la revendication 2, dans lequel la pluralité de groupes d'antennes inclut huit groupes d'antennes (32), chaque groupe incluannt trois antennes dipôles (34) empilées bout à bout.

**4.** Appareil selon l'une quelconque des revendications 1 à 3, dans lequel les premiers moyens (14) pour générer de l'énergie électromagnétique à des fréquences, à une phase et à des niveaux de puissance présélectionnés incluent des moyens diviseurs de puissance (68) pour diviser l'énergie électromagnétique à destination d'une pluralité de voies, une pluralité de moyens amplificateurs de puissance (50) connectés aux moyens diviseurs de puissance (68) pour fixer le gain en puissance de chaque voie, et une pluralité correspondante de moyens de déphasage (48) connectés aux moyens diviseurs de puissance (68) pour fixer la puissance et la phase de chaque voie.

**5.** Appareil selon la revendication 4, dans lequel la pluralité de moyens amplificateurs de puissance (58) des premiers moyens (14) est un amplificateur à quatre voies, les moyens diviseurs de puissance (68) sont un diviseur de puissance à quatre voies, et incluant, en outre, un diviseur de puissance (52) connecté à chaque amplificateur (50) pour diviser la puissance entre les groupes d'antennes (32) et les troisièmes moyens (22).

6. Appareil selon la revendication 4, dans lequel la pluralité de moyens amplificateurs de puissance (50) des premiers moyens (14) est un amplificateur à huit voies et les moyens diviseurs de puissance (68) sont des moyens diviseurs de puissance à huit voies connectés à l'amplificateur à huit voies.

7. Appareil selon la revendication 4, dans lequel la pluralité de moyens amplificateurs de puissance (50) des premiers moyens (14) est un amplificateur à vingt-quatre voies et les moyens diviseurs de puissance sont un diviseur de puissance à vingt-quatre voies (68).

8. Appareil selon la revendication 4, dans lequel la pluralité de moyens amplificateurs de puissance (50) est un amplificateur à seize voies, les moyens diviseurs de puissance (68) sont un diviseur de puissance à seize voies, et dans lequel huit groupes d'antennes (32) des troisièmes moyens (22) et incluent chacun trois antennes dipôles (34) empilées bout à bout et dans lequel huit amplificateurs (50) de l'amplificateur à seize voies sont connectés directement à l'antenne située au centre du groupe de huit antennes (32), et les huit amplificateurs restants sont connectés à huit moyens diviseurs de puissance bidirectionnels (64), chacun des moyens diviseurs de puissance bidirectionnels étant connecté aux dipôles extrêmes opposés d'un groupe d'antennes (32), et la phase des antennes extrêmes opposées de chacun des groupes d'antennes différant de la phase des antennes situées au centre de ce groupe pour l'ajustement de la taille du point chauffant selon la direction de l'axe long.

9. Appareil selon l'une quelconque des revendications 1 à 8, incluant, en outre, des moyens radiométriques (26) connectés aux troisièmes moyens (22) pour mesurer l'énergie rayonnant de la partie de corps (62) entourée par les troisièmes moyens afin de déterminer, de manière non invasive, la température du point chauffant formant cible et de son milieu environnant.

10. Appareil d'hyperthermie selon la revendication 9, dans lequel les moyens radiométriques incluent des moyens de commutation (20) connectés entre les seconds (18) et troisièmes (22) moyens pour connecter sélectivement les troisièmes moyens (22) aux seconds moyens (18) afin de recevoir l'énergie électromagnétique et aux moyens radiométriques (26) afin de recevoir l'énergie rayonnant de la partie de corps (62) par l'intermédiaire des troisièmes moyens (22), un ensemble déphaseur d'énergie rayonnante (21) connecté aux moyens de commutation (20) pour ajuster la phase de l'énergie rayonnante reçue, un mélangeur (24) connecté à l'ensemble déphaseur d'énergie rayonnante (21) pour mélanger les signaux d'énergie rayonnante reçus, et un radiomètre (26) pour mesurer les signaux d'énergie rayonnante délivrés par le mélangeur (24) de manière à déterminer la température du point chauffant formant cible.

11. Appareil selon l'une quelconque des revendications 1 à 10, dans lequel les troisièmes moyens (22) incluent une pluralité de groupes d'antennes (32) montés sur un tube (54) en matériau diélectrique, et incluant, en outre, un capteur de température (28) conçu pour être inséré dans la zone chauffée afin de détecter la température de la cible chauffée, et des moyens de contrôle de température (30) couplés au capteur de température (28) pour contrôler la température détectée du capteur de température et pour transmettre des signaux aux premiers moyens (14) afin de commander le fonctionnement des premiers moyens.

12. Appareil selon l'une quelconque des revendications 1 à 11, incluant des moyens de commande (12) connectés aux premiers moyens (14) pour commander les fréquences, la phase et les niveaux de puissance du signal de sortie des premiers moyens (14).

13. Appareil selon l'une quelconque des revendications 9 à 12, incluant, en outre, des moyens de contrôle de température (30) connectés aux troisièmes moyens (22) pour contrôler la température du point chauffant formant cible et des moyens de commande (12) connectés aux moyens radiométriques (26) et aux moyens de contrôle de température et aux premiers moyens (14) pour commander le signal de sortie de phase et niveau de puissance des premiers moyens (14) en réponse aux signaux de sortie des moyens radiométriques (26) et des moyens de contrôle de température.

14. Appareil selon l'une quelconque des revendications 1 à 13, dans lequel les seconds moyens (18) incluent des moyens transmetteurs d'énergie électromagnétique (36, 38, 40, 42) transmettant l'énergie électromagnétique reçue en provenance des premiers moyens (14) aux troisièmes moyens (22), les moyens desdits moyens conducteurs d'énergie électromagnétique (36,...42) pour ajuster les phases de l'énergie électromagnétique distribuée à des parties des troisièmes moyens (22) incluant des moyens diviseurs d'énergie (36) pour diviser l'énergie reçue en provenance des premiers moyens (14), et une pluralité de conducteurs d'énergie électromagnétique (36, 40, 42) connectés entre les moyens diviseurs d'énergie (36) et les troisièmes moyens (22) et ayant des longueurs présélectionnées pour produire des signaux de sortie d'énergie ayant des phases correspondant aux longueurs de la pluralité de conducteurs d'énergie électromagnétique (36, 40, 42) en vue de leur utilisation par les troisièmes moyens (22) afin de focaliser l'énergie électromagnétique de manière à constituer un point chauffant formant cible.

15. Appareil selon la revendication 14, dans lequel un module déphaseur (46) est connecté aux moyens diviseurs d'énergie (36, 44) au milieu d'au moins l'un de la pluralité de conducteurs d'énergie électromagnétique (38, 40, 42) pour fixer la phase du signal de sortie d'énergie dudit au moins l'un de la pluralité de conducteurs d'énergie électromagnétique.

FIGURE 1

3D CYLINDRICAL DIPOLE ARRAY

FIGURE 2

FIGURE 3

FIGURE 4

Figure 5

FIGURE 6

FIGURE 7

FIGURE 8

FIGURE 9

FIGURE 10

FIGURE 11

Figure 12

EP 0 612 260 B1